# EUROPEAN PATENT SPECIFICATION

(11) **EP 0 885 006 B1**
(45) Date of publication and mention of the grant of the patent: **18.08.2004**
(21) Application number: 97903257.0
(22) Date of filing: 12.02.1997
(51) Int. Cl.: A61K 31/704

(54) **PHARMACEUTICAL COMPOSITION ENABLING TO INHIBIT CANCER METASTASIS FORMATION CONTAINING N-ACETYL-CYSTEINE AND DOXORUBICIN**
N-ACETYLCYSTEIN UND BOXONUBICIN ENTHALTENDE PHARMAZEUTISCHE ZUSAMMENSETZUNG ZUR HEMMUNG DER KREBSMETASTASIERUNG
COMPOSITION PHARMACEUTIQUE PERMETTANT D'INHIBER LA FORMATION DE METASTASES CANCEREUSES ET CONTENANT N-ACETYL-CYSTEINE ET DOXORUBICINE

(30) Priority: 14.02.1996 IT MI960277
(43) Date of publication of application: 23.12.1998
(73) Proprietor: ZAMBON GROUP S.p.A., 36100 Vicenza (IT)
(72) Inventor: DE FLORA, Silvio, I-16167 Genova Nervi (IT); ALBINI, Adriana, I-16134 Genova (IT)
(74) Representative: Longoni, Alessandra
(86) International application number: PCT/EP1997/000627
(87) International publication number: WO 1997/029759

(56) References cited:
- US-A- 4 331 648
- US-A- 4 873 088
- INTERNATIONAL JOURNAL OF CANCER, vol. 67, no. 6, 17 September 1996, pages 842-848, XP000675221 S. DE FLORA ET AL.: "SYNERGISM BETWEEN N-ACETYLCYSTEINE AND DOXORUBICIN IN THE PREVENTION OF TUMORIGENICITY AND METASTASIS"
- CLINICAL & EXPERIMENTAL METASTASIS, vol. 14, no. S1, September 1996, page 24 XP000675205 A. ALBINI ET AL.: "PREVENTION OF TUMORIGENICITY AND METASTASIS IN MURINE MODELS BY N-ACETYLCYSTEINE AND SYNERGISM WITH DOXORUBICIN"
- J. CLIN. INVEST., vol. 68, no. 4, 1981, pages 1053-1064, XP000067349 J.H. DOROSHOW ET AL.: "PREVENTION OF DOXORUBICIN CARDIAC TOXICITY IN THE MOUSE BY N-ACETYLCYSTEINE" cited in the application
- SEMINARS IN ONCOLOGY, vol. 10, no. 1, 1983, pages 53-55, XP000675353 C. MYERS ET AL.: "A RANDOMIZED CONTROLLED TRIAL ASSESSING THE PREVENTION OF DOXORUBICIN CARDIOMYOPATHY BY N-ACETYLCYSTEINE"
- SEMINARS IN ONCOLOGY, vol. 10, no. 1, 1983, pages 29-34, XP000675354 R.D. OLSON ET AL.: "INFLUENCE OF N-ACETYLCYSTEINE ON THE ANTITUMOR ACTIVITY OF DOXORUBICIN"
- TOXICOLOGY AND APPLIED PHARMACOLOGY, vol. 54, no. 1, 1980, pages 168-175, XP000675334 R.W. FREEMAN ET AL.: "EFFECT OF SULFHYDRYL-CONTAINING COMPOUNDS ON THE ANTITUMOR EFFECTS OF ADRIAMYCIN"
- CANCER RESEARCH, vol. 50, no. 7, 1990, pages 2018-2021, XP000675346 F. IMAMURA ET AL.: "POTENTIATION OF INVASIVE CAPACITY OF RAT ASCITES HEPATOMA CELLS BY ADRIAMYCIN"
- MINNESOTA MEDICAL ASSOCIATION, vol. 67, no. 6, 1984, pages 333-335, XP000675351 D.T. KIANG ET AL.: "BREAST CANCER TODAY"
- INTERNATIONAL JOURNAL OF CANCER, vol. 61, no. 1, 1995, pages 121-129, XP000675336 A. ALBINI ET AL.: "INHIBITION OF INVASION, GELATINASE ACTIVITY, TUMOR TAKE AND METASTASIS OF MALIGNANT CELLS BY N-ACETYLCYSTEINE" cited in the application

## Description

The present invention relates to a pharmaceutical composition with antitumor activity and more in particular it relates to an association between Doxorubicin and N-acetyl-cysteine particularly useful to combact spreading of cancer metastases.

N-acetyl-cysteine (The Merck Index, 11th Ed., N. 82, page 14) (hereinafter shortly referred to as NAC) is a well-known drug precursor of reduced glutathione (GSH) to which, in the latest years, useful properties in the prevention of tumors were acknowledged [De Flora S. et al., J. Cell. Biochem. Suppl., 22, 33 (1995)].

NAC is in clinical trial phase for this latter use [Kelloff G.J. et al., J. Cell. Biochem. Suppl., 20, 63 (1994)].

Recent studies have also evidenced how NAC can exert protective mechanisms also in the more advanced stages of cancerogenesis (invasion and metastasis) [Albini A. et al., Int. J. Cancer, 61, 121 (1995)].

Doxorubicin (The Merck Index, 11th Ed. N. 3428, page 540) (hereinafter shortly referred to as DOX) is an anthracyclinic antibiotic endowed with antitumor activity and extensively used in therapy.

The use of DOX is limited by the fact that it may cause potentially lethal congestive cardiomyopathys.

It has been evidenced how NAC enables to attenuate the cardiotoxicity of DOX in mice without decreasing its therapeutic usefulness [Doroshow J.H. et al., J. Clin. Invest., 68, 1053 (1981); US-A-4 331 648 (JR. MYERS ET AL.) 25 May 1982 ; SEMINARS IN ONCOLOGY, vol. 10, no. 1, 1983, pages 53-55; C. MYERS ET AL.; SEMINARS IN ONCOLOGY, vol. 10, no. 1, 1983, pages 29-34, R.D. OLSON ET AL; TOXICOLOGY AND APPLIED PHARMACOLOGY, vol. 54, no. 1, 1980, pages 168-175, R.W. FREEMAN ET AL.; MINNESOTA MEDICAL ASSOCIATION, vol. 67, no. 6, 1984, pages 333-335, D.T. KIANG ET AL.]

"U.S. Patent No. 4,873,088 (Liposome Technology Inc.) describes a liposome formulation in which DOX is associated to (a) compound able to reduce the toxicity of DOX (including NAC) or (b) a compound able to induce host defence (NAC is not included).

Also in this case the use of NAC is that of decreasing the cardiotoxicity of DOX.

The publication "Cancer Research, 50, 2018 (1990)" reports the in vitro observation that DOX potentiate the invasive capacity of the W1 cells. The increase of the invasive capacity is antagonised by pre-treatment with NAC".

We have now surprisingly found that between NAC and DOX exists a synergism in the inhibition of cancer metastasis formation.

It is therefore object of the present invention a pharmaceutical composition containing N-acetyl-cysteine and Doxorubicin in amounts effective to give a synergistic effect in the inhibition of cancer metastasis formation.

It is a second object of the present invention a kit comprising N-acetyl-cysteine and

Doxorubicin in amounts synergistically effective in the inhibition of cancer metastasis formation as well as a suitable medium for the administration thereof.

It is a further object of the present invention a process for the preparation of a pharmaceutical composition containing N-acetyl-cysteine and Doxorubicin for the inhibition of cancer metastasis formation.

The inhibitory activity of cancer metastasis formation was evaluated in laboratory animals in 4 studies of experimental metastases and 4 studies of tumorigenicity and "spontaneous" metastases.

The detail of the experiments is reported in example 1.

In the experiments carried out, in general, the treatment with DOX alone did not reduce significantly the number of metastases with respect to controls, unless at a dose of 10 mg/kg body weight injected i.v. (experimental metastases) and at a dose of 2 mg/kg body weight injected i.p. (tumorigenicity and spontaneous metastases).

In experimental metastasis assays NAC alone reduced significantly the number of metastases only when administered jointly with the infesting cancer cells whereas showed significant protective effects in all the experiments of tumorigenicity and spontaneous metastases.

A remarkable synergistic effect was instead noticed in both kinds of studies, while administering both NAC and DOX, and this effect results to be particularly high when DOX is administered by intravenous route.

NAC may be administered either orally or by intravenous route also in conjunction with DOX.

In the practical aspects of the treatment, NAC and DOX are preferably administered jointly in a suitable pharmaceutical composition.

Examples of specific pharmaceutical forms suitable for the joint administration of the two drugs are the injectable solutions optionally preparable at the time of use.

The compositions may contain a biologically compatible inert solvent, preferably water itself, a buffering and optionally other additives.

The amounts of NAC and DOX per single dose will be modulated as a function of the conditions of the patient, of the type of tumor, of the seriousness and stage of the disease.

NAC is used in doses comprised between 100 mg, to 6 g in a single or more administrations.

The dose of DOX will be the one sufficient to exert a synergistic effect with NAC and will be in the range of 1-10 mg/kg body weight, also in one or more administrations.

Each single dose contains 1-50 mg of DOX.

The extemporaneous preparation of the injectable solution may be carried out by mixing two suitable solutions of the drugs when used.

A suitable kit might comprise the solutions of the two drugs, a suitable medium for their mixing and an injection syringe. Alternatively the syringe might contain one of the two solutions and the mixing might occur inside the syringe itself

Also alternatively, with the aim of reducing to a minimum the operations to be carried out by medical staff, because of the well-known dangerousness of DOX, the kit might contain a pre-filled syringe with the solution to inject.

In the hypothesis wherein the antitumor therapy comprises the use of DOX in conjunction with other drugs with anti-tumor activity such as cyclophosphamide, cisplatin, bleomycin, particular contra-indications in the contemporaneous administration of NAC too are not foreseen.

It will be obviously opportune to verify the chemical compatibility of NAC with said drugs in case it is intended to give NAC in conjunction with them into a single pharmaceutical form, together with DOX.

With the aim of better illustrating the present invention the following examples are now given.

### Example 1

### Materials and methods

### Drugs

DOX was used in the form of commercial injectable product, containing 10 mg of drug per vial (Adriblastina®, Farmitalia - Carlo Erba, Milano, Italia), to be dissolved in distilled water when injected. In the experiments wherein the oral administration was provided, NAC was used in the form of commercial product containing 200 mg of NAC per dosage (Fluimucil®, Zambon Italia, Vicenza), directly dissolved in the drinking water of mice. In the experiments wherein the parenteral (i.p. or i.v.) administration of NAC was foreseen, a 98% pure laboratory reagent (Sigma Chemical Co., St. Louis, MO, U.S.A.) was used, dissolved in a phosphate buffer (PBS), pH 7.4, and furtherly brought to neutrality with NaOH 0.1 N ("spontaneous" metastasis assays) or in NaCl 0.15 M, taking the solution to pH 7.0 (experimental metastasis assays).

### Animals

A total of 385 adult mice was used (Charles River, Calco, Como, Italia) of which 196 female nude (CD-1)BR mice, aged 7 weeks, average weight of 25 g and 189 female C57BL/6 mice aged 6-8 weeks, average weight of 20 g, used in the experimental metastasis assays and in those of "spontaneous" metastases respectively. The (CD-1)BR mice were housed in sterile cages with filtering cover, in a number of 3 animals per cage, at a room temperature of 26-28°C, with a relative humidity of 55%, a ventilation accounting for 12-15 air/h renewals and a 12 hours day/night cycle; the C57BL/6 mice were stabulated in a number of 5 animals per cage, at a temperature of 25-27°C, with a relative humidity of 50% and a 12 hours day/night cycle. The (CD-1)BR mice received a special diet sterilized under vacuum (Mucedola S.r.L, Settimo Milanese, Milano, Italia) and sterilized drinking water; the C57BL/6 mice received a standard rodent diet (MIL Topi e Ratti, Morini, S. Polo d'Enza, Reggio Emilia, Italia) and drinking water ad libitum throughout the duration of the experiments. Within the experiments wherein the sacrifice of mice was foreseen at a scheduled time, killing was carried out by means of ethyl ether anaesthesia and subsequent cervical dislocation. The stabulation and all the animal treatments were carried out in accordance with the national and Community guidelines (D.L. 27.01.1992 N. 116; 86/609/EEC Directive).

### Experimental metastasis assays

In this kind of assay cancer cells were injected in the lateral tail vein, in order to avoid the formation of primary tumors and directly provide the spread of metastases in the lungs. B16-F10 murine melanoma cells were used, resuspended in serum-free Eagle's MEM medium, and injected i.v. in a volume of 100 µl (5 x 10⁴ cells/mouse). As reported in Table 1, wherein also the number of mice included in each experimental group is indicated, 4 groups were always involved in each experiment, namely a control group (either untreated mice or treated with NaCl 0.15M), a group of mice treated with DOX only, a group of mice treated with NAC only and a group of mice treated with the two combined drugs.

In particular, in experiment #1 DOX was injected in a single dose (5 mg/kg body weight) by i.p. route 24 hours after injection of cancer cells, whereas NAC was administered every day with drinking water, at a calculated dose of 2 g/kg body weight, starting 3 days before injection of cancer cells and continuing throughout the duration of the experiment.

In experiment #2 DOX was injected in a single dose (10 mg/kg body weight) by i.v. route 3 days after injection of cancer cells, whereas NAC was administered daily by i.p. route (1 g/kg body weight), starting 8 hours before injection of cancer cells and continuing for 8 days.

In experiment #3 both DOX and NAC were injected by i.v. route 24 hours after injection of cancer cells, at doses of 1 mg/kg body weight and of 6.5 mg/kg body weight, respectively; in the combined treatment therefore the two drugs were injected after being mixed into the same syringe.

In experiment #4, wherein the control and DOX groups were the same as experiment #3, NAC was injected by i.v. route together with cancer cells, dissolved in the culture medium at a concentration of 10 mM.

Within each experiment, all mice were killed at the timing the "natural" death of the first animal in which the presence of lung metastases was observed occurred, namely at a timing of 25 days (experiment #1), 27 days (experiments #3 and #4) and 29 days (experiment #2) from injection of cancer cells. At these timing the necropsy was carried out. The lungs were removed, rinsed in PBS and fixed with buffered formalin.

The total number of visible surface lung tumors was evaluated with the aid of a dissecting microscope.

### Tumorigenicity and "spontaneous" metastasis assays

In this kind of experiment, B16-BL6 murine melanoma cells were injected s.c. in the footpad of the right hind leg (5 x 10⁵ cells/mouse in experiment #5, 2 x 10⁵ cells/mouse in experiments #6, #7 and #8).

With such a technique a primary tumor is formed in the side of injection, from which starts the spread of metastases to the lungs.

Also in these experiments 4 groups were taken into consideration. In all the experiments NAC was administered with drinking water, at a dose of 2 g/kg body weight, starting 48-72 hours before injection of cancer cells and continuing until the end of each experiment. In experiment #5 DOX was administered by i.p. route (2 mg/kg body weight), 24 hours after injection of cancer cells. In experiments #6, #7 and #8 DOX was instead administered by i.v. route (10 mg/kg body weight), 24 hours (#6 and #7) or 7 days (#8) after injection of cancer cells.

In experiments #5, #7 and #8 mice were maintained until spontaneous death, so as to have indications also about the effect of treatment on survival. Both the primary tumors (frequency and weight) and the lung metastases (frequency and number) were therefore evaluated at the time of death.

In experiment #6 instead, 4 weeks after injection of the cancer cells, the leg carrying the primary tumor was removed. Further 4 weeks later the animals were killed, the tumors which gave recurrencies in the leg stump were excised and weighed, and the lung metastases were scored.

### Statistical analysis

The significance of the possible variations in the investigated parameters in mice individually treated with either DOX or NAC as compared to controls was evaluated in each experiment. Moreover the effects of the combined treatment were evaluated with respect both to controls and to each single treatment.

The significance of the variations in the frequency of mice bearing primary tumor, local recurrency or lung metastases was evaluated by Fisher's exact test. The significance of the variations in the mean weight of the primary tumors and of local recurrencies, in the mean number of lung metastases and in the mean days of survival was evaluated either by Student's t-test or by nonparametric Mann-Whithney's U test. In experiments #7 and #8 moreover the survival of mice within each experimental group was compared on a daily basis by χ² analysis.

### Results

### Experimental metastases

Four experiments were carried out, two of which partially overlapping (#3 and #4) in order to evaluate the individual and combined effects of NAC and DOX on the induction of experimental metastases. In all the experiments 5 x 10⁴ B16-F10 murine melanoma cells were injected i.v. in female nude (CD-1)BR mice. When the first spontaneous death occurred, all the animals within the experiment were sacrificed and the lung metastases were counted. The results of these experiments are summarized in Table 1.

In experiment #1 DOX was administered in a single injection i.p. (5 mg/kg body weight) 24 hours after injection of cancer cells. NAC was administered daily with drinking water (2 g/kg body weight), starting 3 days before injection of cancer cells and continuing until the end of the experiment. Under these conditions, compared to control mice injected with cancer cells but without further treatments, DOX and NAC decreased the multiplicity of lung metastases 4.1 fold and 1.3 fold respectively. Such a decrease nevertheless did not result as statistically significant. The combined treatment decreased lung metastases 5 fold, with a difference compared to controls close to the statistical significance threshold.

In experiment #2 DOX was administered in a single injection i.v. (10 mg/kg body weight) 3 days after injection of cancer cells. This treatment caused a significant decrease either in the frequency of mice affected by metastases (from 66.7% to 33.3%) or in the mean number of metastases per animal, which was decreased 17.4 fold. NAC, administered daily i.p. (1 g/kg body weight), starting 8 hours before injection of cancer cells and continuing for the subsequent 8 days, did not affect the frequency of animals with metastases and even increased their multiplicity, but not to a significant extent. The combination of the two drugs produced dramatic protective effects. In fact, the frequency in mice with metastases dropped to 6.7%, a decrease which resulted to be not only highly significant with respect either to controls or to mice treated with NAC only, but which also approached the significance threshold with respect to mice treated with DOX only.

Due to the strong effect noticed with DOX in experiment #2, in the subsequent experiments (#3 and #4) this drug was administered i.v. at a 10 times lower dose (1 mg/kg body weight), 24 hours after injection of cancer cells. Under these conditions DOX resulted almost ineffective at all in modulating the induction of lung metastases.

In experiment #3 NAC was administered i.v. at a low dose (6.4 mg/kg body weight) 24 hours after injection of cancer cells, causing an appreciable but not significant decrease (2.8 fold) of metastases. The combination of the two drugs, mixed together in the same injection i.v. 24 hours after cancer cells, decreased the frequency in mice affected by metastases (from 63.6% to 36.4%, not significant) and reduced drastically the number of metastases, and precisely 61.3 fold with respect to controls (significant), 48.7 fold with respect to mice treated with DOX only (significant) and 32.8 fold with respect to mice treated with NAC only (not significant).

In experiment #4 NAC was dissolved in concentration 10 mM in cancer cells culture medium. This caused a significant decrease of metastases (14.7 fold) with respect to controls injected with untreated cancer cells.

The subsequent injection of DOX after 24 hours which, as already mentioned, was per se ineffective, produced a remarkable synergistic effect. In fact the decrease in the number of metastases was equal to 61.3 fold compared to controls, 48.7 fold compared to DOX alone and 4.2 fold compared to NAC alone. All these differences resulted to be statistically significant.

### Tumorigenicity and "spontaneous" metastases

Four additional experiments, two of which partially overlapping (#7 and #8) were designed in order to evaluate the effects of NAC and DOX, individually or in combination, on the formation of local primary tumors and on the subsequent spread of lung metastases. Female C57BL/6 mice received a single injection s.c. of 2-5 x 10⁵ cells/mouse in the footpad of the right hind leg. In three experiments (#5, #7 and #8) the animals were kept until spontaneous death, thereafter the weight of primary tumors and the number of metastases were recorded. In experiment #6 the leg was removed 4 weeks after injection of cancer cells and the primary tumor was excised and weighed. After further 4 weeks all the animals were sacrificed and lung metastases were scored. Moreover, within a certain number of animals local recurrencies at the stump of the paw were observed.

Experiment #5 was a pilot assay with a limited number of animals. DOX was administered i.p. (2 mg/kg body weight) 24 hours after injection of cancer cells and NAC was administered with drinking water (2 g/kg body weight) starting 48 hours before injection of cancer cells and continuing until the end of the experiment. Mice survival was not affected by these treatments. Injection of DOX did not decrease to a significant extent the weight of primary tumors, whereas it significantly decreased (6 fold) the number of lung metastases. The weight of primary tumors was more than halved in mice treated with NAC only, decrease which nevertheless did not result statistically significant, whereas the number of metastases resulted significantly lower (7.2 fold) than in controls. The combination of the treatments with DOX and NAC was very effective to inhibit to a significant extent the formation either of primary tumors (4 fold) or lung metastases (10.7 fold).

In experiment #6 the treatments were as described in experiment #5, except that the dose of DOX was raised to 10 mg/kg body weight. The weight of primary tumors 4 weeks after injection of cancer cells resulted quite low and not affected by single treatments with DOX or NAC. The combined treatment instead produced a significant and marked inhibition of the frequency in mice affected by tumor and even more in the weight of primary tumors, not only with respect to controls but even with respect to each of the two pharmacological individual treatments. Similarly, both frequency and weight of local recurrencies resulted lower in mice which received the combined treatment than in control mice and mice treated with the two drugs individually.

Really none of the 12 mice which received the combined treatment developed lung metastases, this determined a significant drop of their frequency as compared to each of the other three groups and to their number as compared to controls and to mice treated with DOX only. The difference with mice treated with NAC only did not reach instead the threshold of statistical significance because of a 1.6 fold decrease (not significant) in the number of lung metastases in mice treated with this thiol.

NAC p.o. (2 g/kg body weight) enhanced remarkably the survival of mice in experiments #7 and #8, as assessed both by evaluating the mean survival time compared to controls (Table 2) and by comparing daily the survival curves in these two groups. To a minor extent, also DOX i.v. (10 mg/kg body weight) prolonged survival when administered 24 hours after injection of cancer cells. This treatment determined a significant amelioration of the survival curve, expecially within the first 50 days, and enhanced significantly the mean survival time (Table 2, experiment #7). The treatment with DOX 7 days after injection of cancer cells instead did not affect the survival curves nor the mean survival time (Table 2, experiment #8). The combined treatment with NAC and DOX did not furtherly enhance survival as compared to the single treatment with NAC. Nevertheless it is noteworthy that the combined treatments improved survival with respect to the single treatments with DOX, administered 24 hours or 7 days after injection of cancer cells. It is also noteworthy that the combination of NAC with DOX (after 7 days) enhanced the survival time as compared to controls, whereas the single treatment with DOX resulted ineffective (Table 2, experiment #8).

Within the comparisons among the examinated groups in experiments #7 and #8 it is necessary to keep in mind that primary tumors and lung metastases were evaluated in each animal at the time of death. Accordingly, when survival times were significantly enhanced, also similar carcinogenicity data reflect a slower development of cancer cells. All the more reasons, the protective effects are even more important than those inferring from statistical comparisons. In this light, the even non significant decrease in the weight of primary tumors, with respect to controls, are noteworthy within the groups of mice treated with NAC, DOX after 24 hours or with the combination of NAC with each one of the two DOX treatments, since time of death was significantly delayed in all these groups. In the same way, even more remarkable have to be considered the significant inhibitions both in the frequency (4.8 fold) and in the number (2.1 fold) of lung metastases in mice treated p.o. with NAC. A further inhibition in the multiplicity of lung metastases was recorded in mice treated with both NAC and DOX 24 hours after injection of cancer cells. In this group the number of metastases resulted significantly lower when compared not only to controls (12.7 fold) but also to mice treated with DOX only (14.4 fold), and it was remarkable though not statistically significant with respect to mice treated with NAC only (5.9 fold).

### Comments on the synergism between NAC and DOX

Within the four studies on the experimental metastases the treatments varied with respect to the dose of DOX and its administration route (i.p. or i.v.) as well as to the dose ofNAC, its administration route (p.o. or i.v.) and treatment time schedules.

The single treatment with DOX reduced to a significant extent the number of lung metastases only when injected i.v. at a dose of 10 mg/kg body weight.

The single treatment with NAC inhibited significantly such a parameter only when added to the medium werein cancer cells were resuspended at the moment of their injection i.v. in mice. This finding confirms, also from a quantitative point of view, the conclusions of a previous experiment with similar characteristics [Albini A. et al., Int. J. Cancer, 61, 121, (1995)].

The combined treatment with the two drugs caused instead a significant decrease in the lung metastases in all the experiments (only in experiment #1 the decrease was close to the statistical significance threshold). In particular, in experiment #1, the only one wherein DOX was administered i.p., the effectiveness of the combined treatment was approximately additive as compared to the single treatments. In fact the reduction in the number of metastases with respect to untreated controls was 5.0 fold with the two combined drugs, 4.1 fold with DOX only and 1.3 fold with NAC only. In the other 3 experiments, wherein the cytotoxic agent was administered i.v., the combined effect not only resulted more than additive but even more than multiplicative. In fact, the reduction in the number of metastases in mice treated with the two combined drugs, with DOX only and with NAC only was respectively equivalent to 174.3, 17.4 and 0.5 fold in experiment #2, 90.5, 1.3 and 2.8 fold in experiment #3 and 61.3, 1.3 and 14.7 fold in experiment #4.

Within the four tumorigenicity and "spontaneous" metastasis studies NAC was always given p.o. at the same dose (2 g/kg body weight), whereas for DOX were varied the dose (from 2 to 10 mg/kg body weight), administration route (i.p. or i.v.) and time (1 or 7 days after injection of cancer cells).

The individual treatment with DOX decreased to a significant extent the number of lung metastases only in experiment #5, at a dose of 2 mg/kg body weight by i.p. route. The individual treatment with NAC, as a confirmation of the results obtained in a previous study [Albini A. et al., Int. J. Cancer, 61, 121 (1995)], had significant protective effects in experiments wherein mice were kept until spontaneous death (#5, #7/8). In particular, in both experiment #5 and experiment #7/8 (NAC group was common to two experiments), NAC inhibited significantly the number of lung metastases and in experiment #7/8 it also reduced significantly the frequency in mice affected by metastases. Moreover, in experiment #7/8 NAC enhanced remarkably survival times of the animals.

The combined treatment with DOX showed effects more than multiplicative in experiment #6, wherein fixed times were selected to evaluate the primary tumors in all the animals (4 weeks), local recurrencies and lung metastases (8 weeks). In fact, the reduction with respect to controls in mice treated with the two combined drug, with DOX only and with NAC only was equivalent to 7.3, 1.4 and 1.0 fold to what it concerns the weight of primary tumors, 12.5, 1.1 and 0.9 fold to what it concerns the weight of local recurrencies, and ∞ (there was not a single metastasis among mice which received the combined treatment), 0.7 and 1.6 fold to what it concerns the number of lung metastases. In experiment #5 the reductions were equivalent to 4.0, 1.3 and 2.1 fold to what it concerns the weight of primary tumors (effect slightly more than additive), and equivalent to 10.7, 6.0 and 7.2 fold to what it concerns the number of lung metastases (effect slightly less than additive). The association of NAC with DOX administered 7 days after injection of cancer cells (experiment #8), was not particularly effective. The association of NAC with DOX, administered 24 hours after injection of cancer cells (experiment #7), produced instead effects more than multiplicative in reducing lung metastases (12.7, 0.9 and 2.1 fold in mice respectively treated with both drugs, with DOX only and with NAC only).

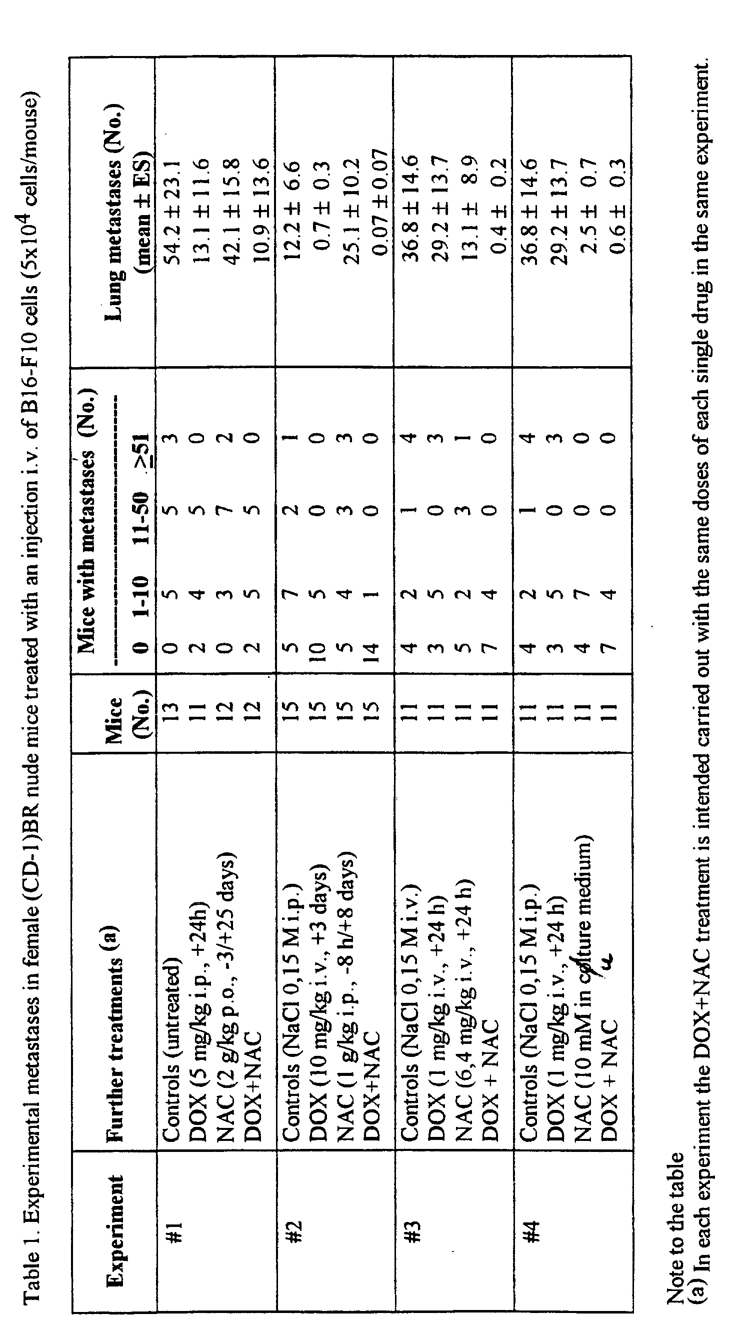

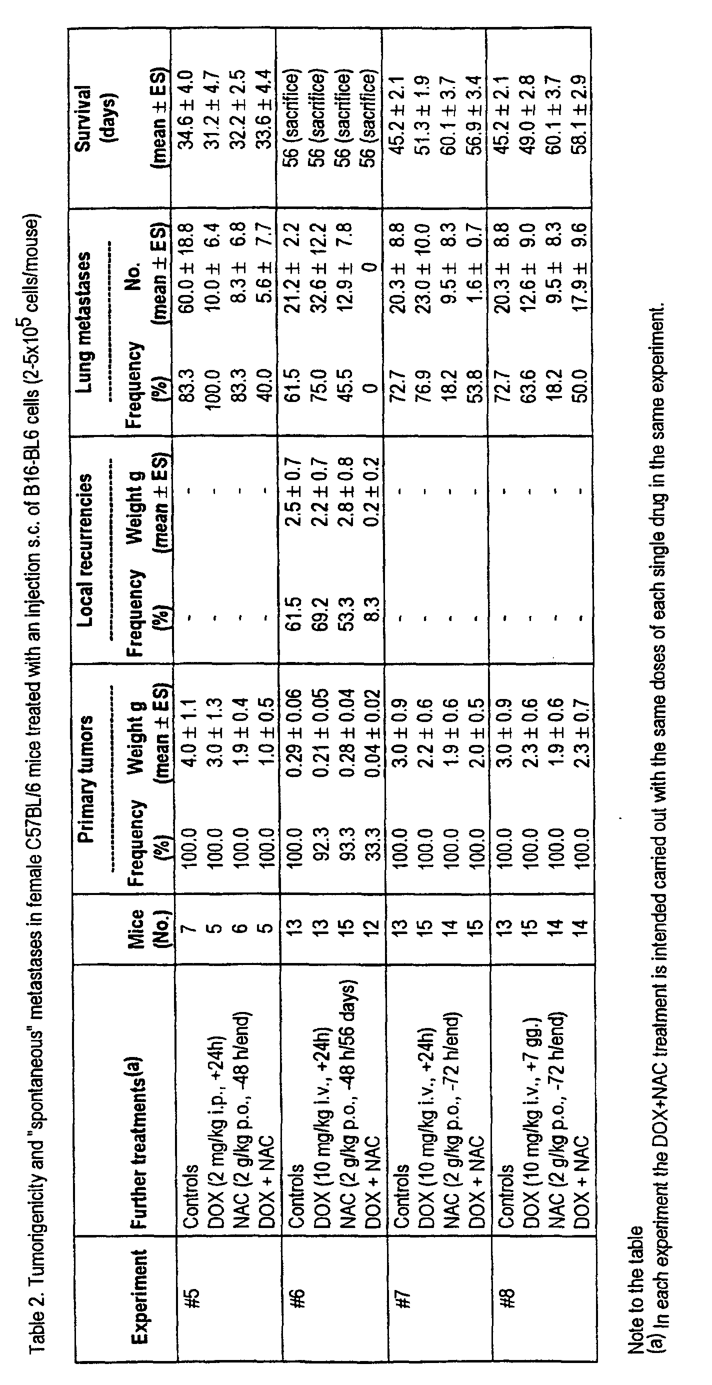

### Example 2

### Injectable solution containing both NAC and DOX

A solution of DOX was prepared by adding distilled water to a vial containing 10 mg of DOX.

A solution of NAC was prepared by dissolving 64 mg of NAC in a phosphate buffer solution at pH 7.4.

The solution was brought to neutrality with NaOH 0.1N.

The two solutions were intaken in a syringe and mixed, affording a ready for use solution.

## Claims

1. A pharmaceutical composition for inhibiting cancer metastases formation containing N-acetyl-cysteine in a dose of 100 mg to 6 g and doxorubicin in a dose from 1 to 50 mg.

## Patentansprüche

1. Arzneimittel zum Hemmen von Krebsmetastasenbildung, das N-Acetylcystein mit einer Dosis von 100 mg bis 6 g und Doxorubicin mit einer Dosis von 1 bis 50 mg enthält.

## Revendications

1. Composition pharmaceutique destinée à l'inhibition de la formation des métastases du cancer contenant du N-acétyle-cystéine sous dose de 100 mg à 6 g par jour et de la doxorubicine sous dose de 1 à 50 mg.
